# EUROPEAN PATENT APPLICATION

(11) **EP 4 850 623 A1**
(43) Date of publication of application: **09.12.2026**
(21) Application number: 26182431.2
(22) Date of filing: 02.06.2026
(51) Int. Cl.: A61B 18/14, A61B 17/00, A61B 18/00

(54) **ASSEMBLY FEATURES AND METHODS FOR ELECTROSURGICAL TOOLS**

(30) Priority: 04.06.2025 US 202563817432 P; 29.05.2026 US 202619691719
(71) Applicant: Arthrex, Inc, Naples, FL 34108 (US)
(72) Inventor: Leach, Coleman, Naples, Florida, 34108 (US); Newell, Christopher, Naples, Florida, 34108 (US); Daley, Robert, Naples, Florida, 34108 (US); Caldwell, Jeremiah D., Naples, Florida, 34108 (US); Hacker, Randall, Naples, Florida, 34108 (US); Taft, Richard J., Naples, Florida, 34108 (US); Krupp, Maxwell, Naples, Florida, 34108 (US); Rivera, Leander, Naples, Florida, 34108 (US)
(74) Representative: Lohr, Jöstingmeier & Partner Patent- und Rechtsanwälte mbB

(57) **Abstract**

An electrosurgical probe includes an elongated probe extending along a longitudinal axis. An inner tube extends through an outer tube. The inner tube forms a lumen extending along a longitudinal axis from a proximal tube portion to a distal tube portion. The proximal tube portion of the outer tube comprises a plurality of mating apertures. A handle portion includes a probe coupling configured to receive the proximal tube portion. The probe coupling comprises a plurality of protrusions that engage the mating apertures.

## Description

### BACKGROUND

The disclosure generally relates to features and methods for an electrosurgical apparatus and, more particularly, to assembly features for an elongated electrosurgical probe. Improvements in various surgical technologies may improve the ability of medical professionals to conduct a wide variety of surgical procedures. As discussed in reference to various features in the following detailed description, the disclosure provides for a variety of components and assembly features for an electrosurgical probe that may improve manufacturability as well as the quality and reliability of the corresponding surgical tools.

### SUMMARY

In various implementations, the disclosure provides for an electrosurgical probe comprising an elongated shaft that may be manipulated by engaging a handle including a user interface. The electrosurgical probe may correspond to a bipolar surgical instrument comprising an electrode tip assembly that may be structurally and conductively connected within a housing forming the handle. As provided in various examples that follow, the elongated probe may include a mating interface having one or more features incorporated in the housing as well as the structure of the elongated probe. Based on these features and several additional assemblies, the disclosure may provide for surgical apparatuses with improved reliability and manufacturability.

In various implementations, the mating interface between the elongated probe and the housing of the handle may include one or more assembly features. The assembly features may include one more mating apertures formed along a proximal end portion of the elongated probe. Further, within the housing, engaging protrusions may be formed by support ribs and aligned with the mating apertures. For example, the elongated probe may be formed by an inner tube that extends within an outer tube. The inner tube may form a lumen through the probe that may be implemented to communicate fluid to or from a surgical site. In various implementations, one or more apertures or slots may be formed through portions of the outer tube and configured to receive the mating protrusions which may provide for axial locating structures within the housing. In addition to the axial alignment, the engagement between the mating protrusions and the corresponding apertures may rotationally orient the elongated probe relative to the handle portion providing a poka-yoke assembly to ensure accurate and reliable assembly of the electrosurgical probe.

In various implementations, the disclosure may provide additional features that may be implemented alone or in combination with the specific aspects of the elongated probe previously introduced. For example, in some implementations, the disclosure may provide for a conductive clip that may be configured to provide a secure conductive connection between a control circuit of the electrosurgical probe and two or more tube sections having different diameters. Further, the disclosure provides for a fluid fitting that may be implemented to securely connect a fluid lumen of the electrosurgical probe that may flexibly be implemented in a variety of electrosurgical devices.

These and other features, objects and advantages of the present disclosure will become apparent upon reading the following description thereof together with reference to the accompanying drawings.

### BRIEF DESCRIPTION OF THE DRAWINGS

FIG. 1 is a projected view of an electrosurgical apparatus demonstrating an elongated probe;
FIG. 2 is a partially exploded assembly view of an electrosurgical apparatus demonstrating various features enclosed within a handle assembly;
FIG. 3 is a partially exploded assembly view of an electrosurgical apparatus demonstrating various features enclosed within a handle assembly;
FIG. 4 is a side cross-sectional view demonstrating an electrode structure of an elongated probe;
FIG. 5A is a projected, partial assembly view demonstrating a conductive connection between active and passive control terminals of a control circuit with active and passive electrodes of an elongated probe;
FIG. 5B is a projected view demonstrating conductive and fluidic connections between the control circuit of the electrosurgical apparatus and one or more control consoles or pumps;
FIG. 6A is a detailed projected view of a conductive clip;
FIG. 6B is a schematic diagram of the conductive clip of FIG. 6A demonstrated in a biased configuration in connection with a tubular or cylindrical conductive body;
FIG. 6C is a schematic diagram of the conductive clip of FIG. 6A demonstrated in a neutral configuration in connection with a tubular or cylindrical conductive body;
FIG. 7A is a side, exploded assembly view demonstrating a self-tapping configuration between an inner tube of the elongated probe and a fluid fitting;
FIG. 7B is a projected view of a fluid fitting demonstrating a cannulated passage;
FIG. 8A is a side view demonstrating a barbed fluid fitting;
FIG. 8B is a side, cross-sectional view demonstrating the barbed fluid fitting of FIG. 8A;
FIG. 8C is a partial cross-sectional view of the fluid fitting of FIGS. 8A and 8B demonstrating a tapered female coupler;
FIG. 9A is a detailed projected view of a conductive clip;
FIG. 9B is a schematic diagram of the conductive clip of FIG. 9A demonstrated in a biased configuration in connection with a tubular or cylindrical conductive body;
FIG. 9C is a schematic diagram of the conductive clip of FIG. 9A demonstrated in a neutral configuration in connection with a tubular or cylindrical conductive body; and
FIG. 10 is a block diagram and environmental view of an electrosurgical system in accordance with the disclosure.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized, and structural and functional changes may be made without departing from the scope of this disclosure.

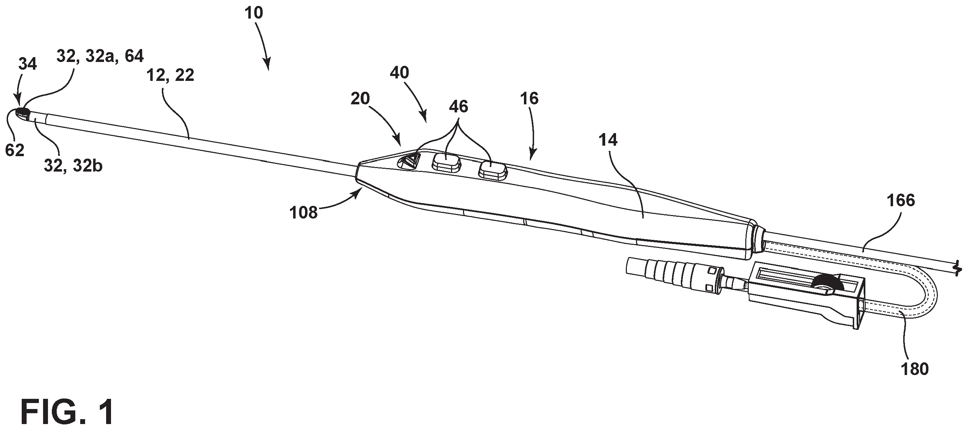

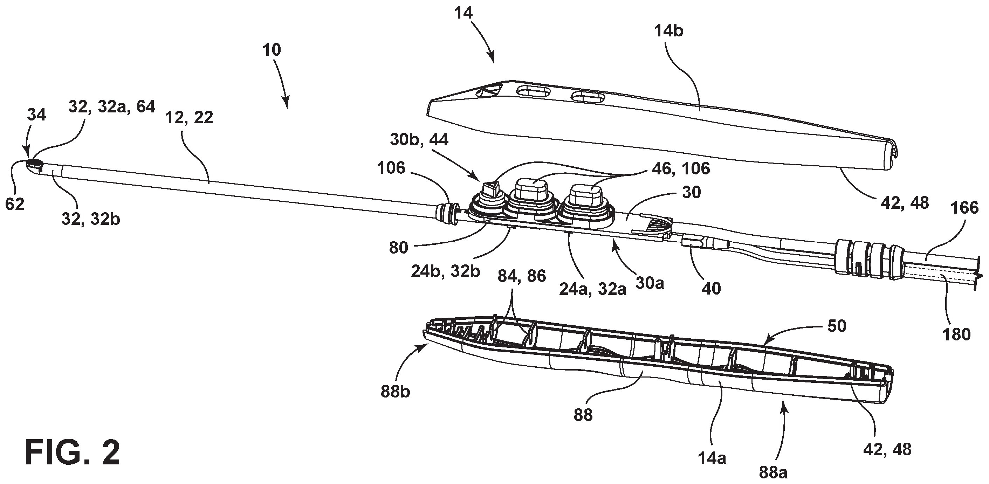

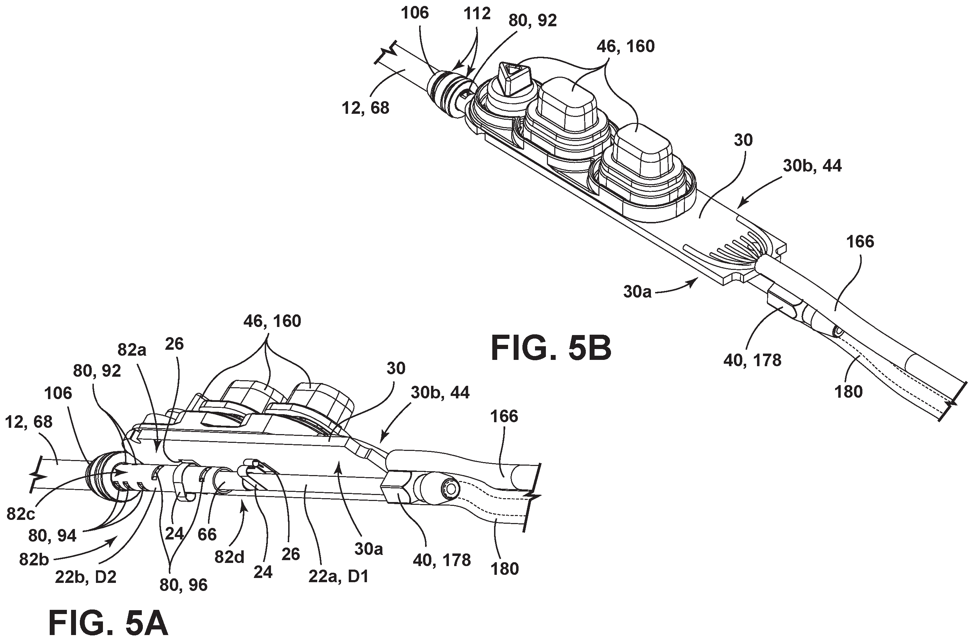

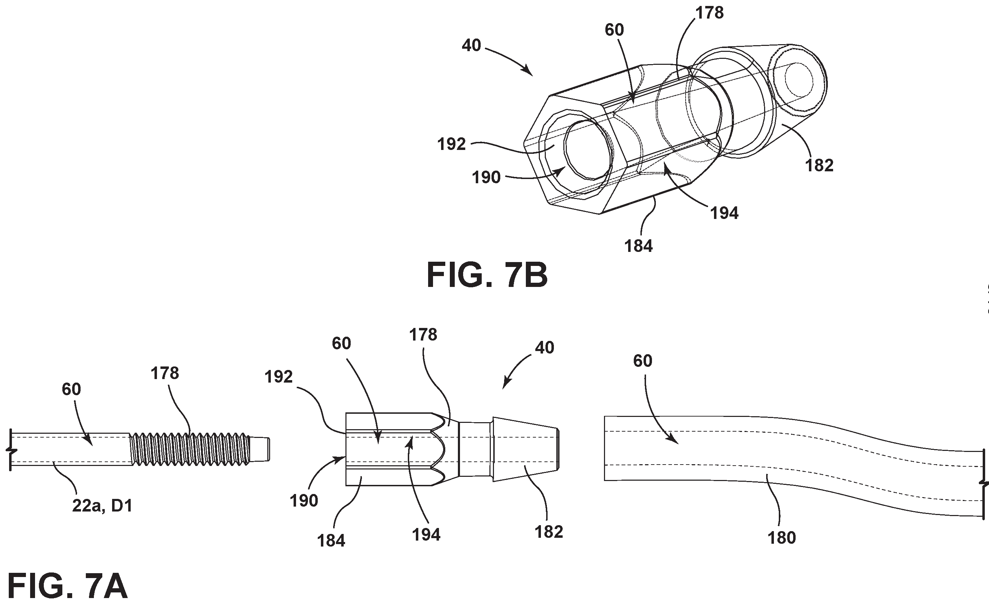

As generally demonstrated in FIG. 1, the disclosure provides for an electrosurgical apparatus 10 comprising an elongated probe 12 in connection with a housing 14 that forms a handle portion 16. As generally demonstrated and discussed in reference to FIGS. 2-4, the electrosurgical apparatus 10 may comprise a mating interface 20 configured to operably couple at least one shaft or tube 22 in connection with the housing 14. As further demonstrated in reference to FIGS. 5-6, the mating interface 20 may include one or more conductive clips 24 that may conductively couple one or more corresponding control terminals 26 of a control circuit 30 to an electrode 32 of the elongated probe 12. In this configuration, the control circuit 30 may effectively conduct control signals via the at least one electrode 32 to effectuate an electrosurgical therapy or resection operation with a distal electrode tip 34 of the elongated probe 12. Finally, as demonstrated and described in reference to FIGS. 7A and 7B, the disclosure may further provide for a fluid fitting 40 for the surgical apparatus 10 that may fluidically couple the at least one tube 22 to a fluid supply or vacuum pump to support a wide variety of applications. As demonstrated in the following examples, each of the disclosed features may be implemented alone or in various combinations to suit various applications for surgical devices.

Referring first to the structural aspects of the mating interface 20, several features are described in reference to FIGS. 1-4. As best demonstrated in FIG. 2, the housing 14 may be formed in a clamshell configuration and include a first side 14a and a second side 14b that may be separated along a parting line 42. In this configuration, several mating features formed by the opposing first and second sides 14a, 14b of the housing 14 may serve to engage corresponding mating apertures formed in the at least one tube 22 to mechanically secure and align the elongated probe 12 to the handle portion 16. The alignment of the elongated probe 21 to the handle portion 16 may align a user interface 44 having one or more user inputs 46 with the handle portion 16 and the electrode tip 34 to securely and accurately assemble the surgical apparatus 10. In various implementations, the opposing first and second sides 14a, 14b of the housing 14 may be fused, ultrasonically welded, adhered, or otherwise affixed along complimentary perimeter edges 48 formed where the first and second sides 14a, 14b of the housing 14 meet. Once connected, the sides 14a, 14b of the clamshell assembly may secure the elongated probe 12 and the control circuit 30 within an interior cavity 50 formed by the housing 14.

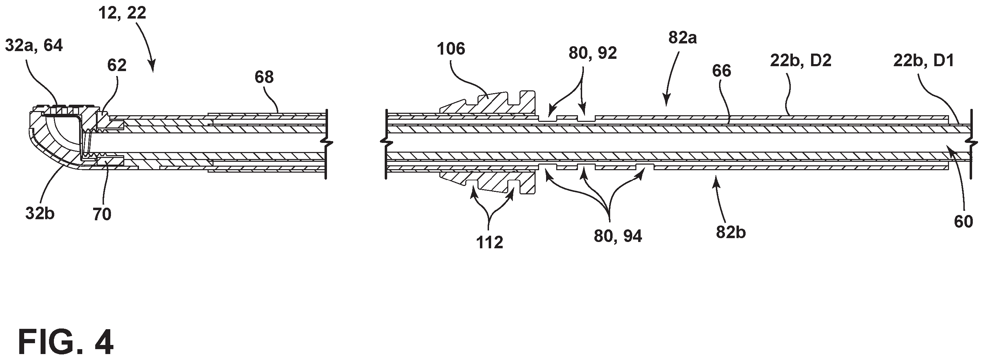

Before further describing several optional details of the mating interface 20, an exemplary operation of surgical apparatus 10 is generally discussed. As shown in FIG. 4, the elongated probe 12 may include at least one electrode 32 formed in part by the at least one tube 22 of the elongated probe 12. The elongated probe 12 may be formed by an inner tube 22a that extends within an outer tube 22b along a longitudinal axis A_{L}. In a bipolar configuration as depicted in the representative examples, the inner tube 22a may form a central lumen 60 that may correspond to a fluid passage configured to aspirate and/or deliver fluid to or from a surgical cavity in a minimal invasive procedure. In various implementations, the tubes 22a, 22b may correspond to an active electrode 32a and a return electrode 32b to support bipolar operation. Accordingly, the control circuit 30 may output control signals to the active electrode 32a and provide a closed circuit via the return electrode 32b that may be bridged across an insulating collar 62. In this configuration, an electrosurgical treatment may be localized at the distal electrode tip 34 in a bipolar configuration. In the example shown, the electrode tip 34 may form an active face 64 configured to deliver an electrosurgical stimulus approximately perpendicular to the longitudinal axis A_{L} of the elongated probe 12. However, it shall be understood that the arrangement of the active face 64 may vary from approximately 0° to 90° or greater depending on the application of the surgical apparatus 10.

Still referring to FIG. 4, the electrodes 32 formed by the tubes 22 may be separated by a first insulating layer 66 that may extend along a length of the elongated probe 12 where the electrodes 32 form an overlapping segment. Additionally, a second insulating layer 68 may extend about an outer face of the outer tube 22b and conductively shield or insulate the outer surface of the return electrode 32b from an operating environment in which the elongated probe 12 is deployed. In this configuration, control signals communicated via the control terminals 26 and the conductive clips 24 (see FIGS. 5A-5B) may be communicated along the active electrode 32a to the distal electrode tip 34, arc over the insulating collar 62 or distal insulator, and return via the return electrode 32b formed by the outer tube 22b. The corresponding operation of the electrodes 32 and the insulating layers 66, 68 may be supported by corresponding conductive and insulating materials, respectively. Accordingly, each of the tubes 22 forming the electrodes 32 may be formed via conductive (e.g., metallic) materials that may efficiently conduct the electrical signals from the control circuit 30 to and from the distal electrode tip 34. The insulating layers 66, 68 may correspond to polymeric, ceramic, or other insulating materials that may conductively isolate the tubes 22 forming the electrodes 32 along the length of the elongated probe 12. Finally, as further demonstrated in FIG. 4, the inner and outer tubes 22 may be mechanically coupled to each other and the electrode tip 34 via an intermediate insulating coupling 70 that may secure the tubes 22 from translating relative to each other along the longitudinal axis A_{L}.

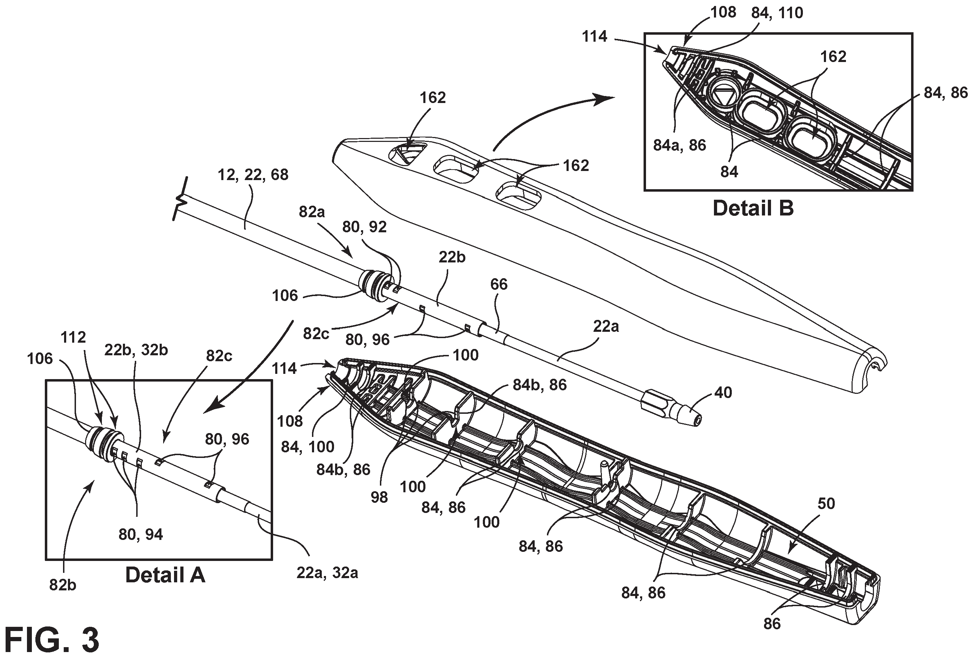

Referring again to FIGS. 2-5, the configuration of the tubes 22 may provide for both the operable coupling of the mating interface 20 as well as the fluidic coupling provided by the central lumen 60 formed within the inner tube 22a. To maintain a fluidic seal within the central lumen 60, a plurality of mating apertures may be formed through the outer tube 22b without penetrating through a wall formed by the inner tube 22a. As best demonstrated in FIGS. 3 and 5A, the mating apertures may be formed along a dorsal surface 82a, ventral surface 82b, as well as lateral surfaces 82c, 82d, and spaced along a length L of the elongated probe 12. Opposite and arranged in correspondence with each of the mating apertures 80, the housing 14 may form a plurality of protrusions 84. As best demonstrated in FIG. 3, one or more of the protrusions 84 may be formed by ribs 86 or baffles that may protrude inward from a shell structure 88 forming the opposing sides 14a, 14b of the housing 14 and into the interior cavity 50. As shown, each of the protrusions 84 may be spaced to align longitudinally with the corresponding mating apertures 80 formed along the length L of the elongated probe 12. In this configuration, the engagement of the sides 14a, 14b of the housing 14 may cause the protrusions 84 to extend into the interior cavity 50 along the length L of the elongated probe 12 and engage the corresponding mating apertures 80, thereby locking the outer tube 22b and the remainder of the attached elongated probe 12 to the handle portion 16 along the longitudinal axis A_{L}.

As previously described, the protrusions 84 may correspond to ribs 86 and extend into the interior cavity 50 to engage the corresponding mating apertures 80. Further, one or more of the protrusions 84 or mating apertures 80 may differ in geometry. For example, the alignment of the protrusions 84 and apertures 80 may differ in shape or direction relative to the longitudinal axis A_{L}. Additionally, the protrusions 84 may engage the mating apertures 80 along different surface 82a-82d about the elongated probe 12. For example, the protrusions 84 may comprise one or more lateral protrusions 84a and/or one or more circumferential protrusions 84b. As shown, a plurality of lateral protrusions 84a may be positioned along a distal end portion 88b of the shell structure 88 opposite a proximal end portion 88a. The lateral protrusions 84a may extend from each of the first side 14a and the second side 14b of the housing 14 and may form a lattice structure configured to engage one or more corresponding dorsal slots 92 and ventral slots 94 that may extend laterally through corresponding dorsal and ventral surfaces 82a, 82b of the outer tube 22b. In this configuration, the lateral protrusions 84 may primarily serve to secure the elongated probe 12 longitudinally within the housing 14.

As best demonstrated in Detail A of FIG. 3, one or more of the dorsal and ventral slots 92, 94 may be asymmetric in shape and/or count. In the example shown, the number of ventral slots 94 may be greater than the number of dorsal slots 92 (e.g., three ventral slots 94 and two dorsal slots 92). Additionally, the corresponding lateral protrusions 84 formed by the first and second sides 14a, 14b of the housing 14 may correspond in number. As shown in FIG. 3, the third proximally positioned lateral protrusion 84 may engage the corresponding third proximally positioned ventral slot 94. However, if rotated about the longitudinal axis 180°, the third proximally positioned lateral protrusion 84, may create an interference, preventing the sides 14a, 14b of the housing 14 from effectively mating. Accordingly, the positioning and configuration of the corresponding components of the mating interface 20 may provide for a poka-yoke assembly preventing a misalignment between the housing 14 and the elongated probe 12. By including a radial asymmetry as a differing number, position, geometry, and/or orientation of the mating apertures 80 and the lateral protrusions 84, the disclosed features may prevent errors in assembly.

Still referring to FIG. 3, the mating interface 20 may further include one or more lateral slots 96 that may be positioned on opposing lateral surfaces 82c, 82d of the outer tube 22b. In an assembled configuration, the lateral slots 96 may receive corresponding side walls 98 formed by opposing sides of a trough 100 and extending along the longitudinal axis A_{L} through the ribs 86. In this configuration, the circumferential protrusions 84 may engage the laterally opposed surface 82c, 82d of the outer tube 22b to further orient and secure the elongated probe 12 within the opposing clamshell sides 14a, 14b of the housing 14.

In addition to the features of the mating interface 20 formed between the outer tube 22b and the housing 14, a distal seal 106 may be secured about a circumference of the elongated probe 12 distal of the lateral protrusions 84 and may be aligned with a nose cone 108 formed by the housing 14. As shown, one or more circumferential ribs 110 may extend into the interior cavity 50 from the shell structure 88 at the distal end portion 88b. In this way, the engagement of the opposing sides 14a, 14b of the housing 14 may cause the circumferential ribs 110 to engage corresponding circumferential receiving channels 112, thereby enclosing a distal opening 114 through which the elongated probe 12 may protrude from the housing 14. In various implementations, the distal seal 106 may correspond to a flexible polymeric material that may be partially compressed within the corresponding portions of the interior cavity 50 between the circumferential ribs 110 to effectively seal the distal end portion 88b of the interior cavity 50.

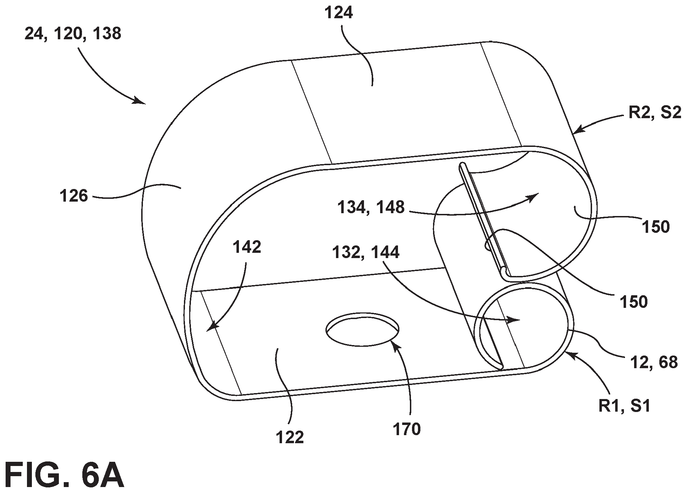

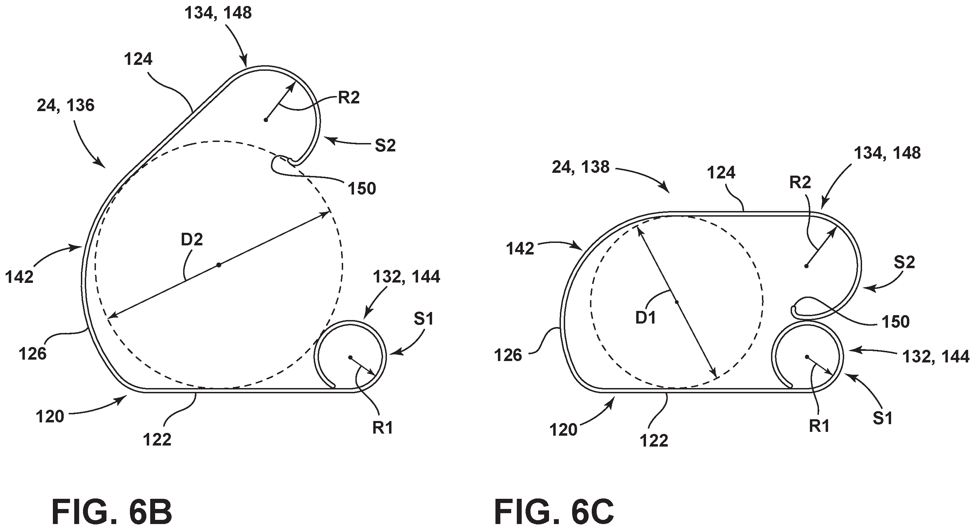

Referring now to FIGS. 5 and 6, additional aspects of the mating interface 20 may ensure that the surgical apparatus 10 can be effectively and accurately assembled. As previously discussed, the surgical apparatus 10 may comprise one or more conductive clips 24 that may conductively connect corresponding control terminals 26 to the active and/or return electrodes 32a, 32b. As best illustrated in FIGS. 5A and 6A, the conductive clips 24 may generally comprise a U-shaped body 120 comprising a thickness T and a width W. A profile shape of the U-shaped body 120 may comprise a first side wall 122 and a second side wall 124 interconnected along a curved spring portion 126. As further discussed later in the description, the side walls 122, 124 may form a corresponding first arc 132 and second arc 134 that may allow the conductive clip 24 to securely, conductively engage conductors (e.g., the inner tube 22a and outer tube 22b) having differing diameters D1, D2 by adjusting from a biased configuration 136 to a neutral configuration 138.

Still referring to FIGS. 5-6 and particularly to FIG. 5A, the at least one conductive clip 24 may include the first conductive clip 24a configured to conductively connect to the active electrode 32a and the second conductive clip 24b configured to conductively connect to the return electrode 32b. However, the same structure of the conductive clip 24 may accommodate each of the diameters D1, D2. In other words, the structure and features of the conductive clip 24 may be implemented to conductively and/or structurally connect with electrode or various electronic components for surgical devices of varying proportions while maintaining common geometry and dimensions. In this way, the conductive clip 24 may limit the need for utilizing different sizes or types of conductive connectors, thereby limiting the complexity of the assembly associated with the surgical apparatus 10.

As illustrated in FIGS. 6B and 6C, the nested relationship between the inner tube 22a forming the active electrode 32a and the outer tube 22b forming the return electrode 32b may cause the diameters D1, D2 to vary significantly. However, various features associated with the conductive clip 24 may ensure that effective circumferential conductive contact may be maintained between contact surfaces 140 of the conductive clip 24 in connection with the differing diameters D1, D2 of the nested inner and outer tubes 22a, 22b. Though not limited to specific examples, the first diameter D1 of the inner tube 22a may vary from approximately 1 mm to 4 mm. The second diameter D2 of the outer tube 22b may vary from approximately 2 mm to 7 mm. Accordingly, the conductive clip 24 may conductively engage tubular bodies with effective diameters having ratios of 3:4, 2:3, 1:2, or 1:3 between the first diameter D1 and the second diameter D2.

As shown in FIG. 6B, the conductive clip 24 is shown in the biased configuration 136. In this configuration, conductive contact with the outer tube 22b may be maintained along a portion of a trough 142 and the side walls 122, 124 of the U-shaped body 120. Further, conductive contact may be secured and maintained by the engagement of a portion of the first arc 132 and/or the second arc 134 at least partially wrapping around and securing the second diameter D2 within the conductive clip 24. As shown, the first arc 132 may comprise a first radius R1 and extend circumferentially to a first arc length S1 that may form a substantially enclosed circle 144. Additionally, the second side wall 124 may form the second arc 134 having a second radius R2 that may be greater than the first radius R1. The second arc 134 may further comprise a second arc length S2 that may be less than the first arc length S1. In this configuration, the second arc length S2 of the second arc 134 may form a substantially open arc shape 146, such that the second arc 134 generally forms an opposing U-shape 148 opposite the curved spring portion 126. Further, a distal end of the second arc 134 may form an engagement tab 150 that may extend laterally from the distal end of the opposing U-shape 148, such that a length of the tab extends approximately along a tangential surface of the second diameter D2 of the outer tube 22b.

As noted, the second radius R2 may be greater than the first radius R1. In various implementations, the relative proportions of the first radius R1 and the second radius R2 may be expressed by a ratio of R1 to R2. For example, the ratio of R1 to R2 may be greater than or equal to one of: 4:5, 3:4, 2:3, 1:2, 1:3, or 1:4. The proportions of each the radii R1, R2 of the arcs may be set based on the corresponding proportions of the diameters D1 and D2 to ensure the conductive clip effectively mates with the tubes 22a, 22b, In this way, the design of the conductive clip 24 may vary to suit a wide range of tubes of similar elongated bodies to provide an effective conductive connection.

As further demonstrated in FIG. 6C, the conductive clip 24 may be implemented in the neutral configuration 138. In this configuration, the curved spring portion 126 may enclose the first diameter D1 of the inner tube 22a. The neutral configuration 138 may still apply a spring force or retention force enclosing the clip 24 about the inner tube 22a along the opposing first and second side walls 122, 124. The relationship and spacing S may be maintained by the first arc 132 abutting the second arc 134. Accordingly, the spacing S between the first and second side walls 122, 124 may be defined by the combined radii R1 and R2 or alternatively, the corresponding diameters associated with the arcs 132, 134. As further demonstrated in FIG. 6C, the U-shaped body 120, the side walls 122, 124, and the first and second arcs 132, 134 may completely enclose the perimeter of the inner tube 22a and the corresponding first diameter D1. Accordingly, the conductive clip 24 may provide for a secure conductive connection between the control terminals 26 of the control circuit 30 with each of the tubes 22 in the biased configuration 136 and the neutral configuration 138.

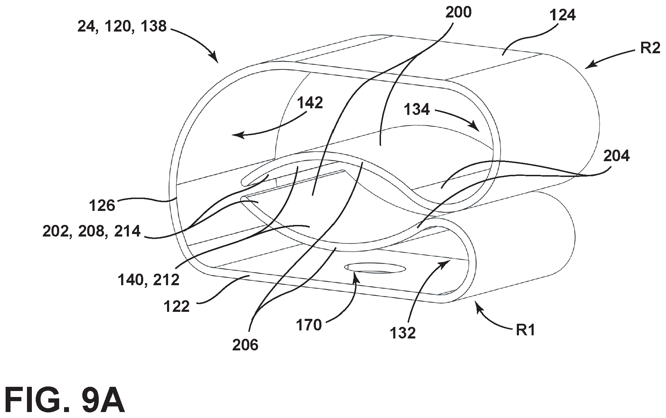

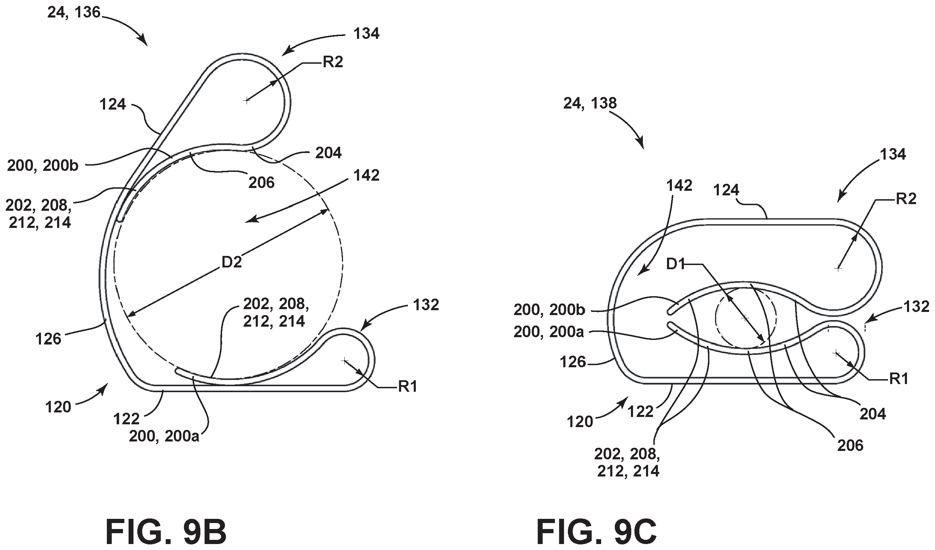

Referring again to FIGS. 5A, 5B, and 6A, instances of the conductive clip 24 are shown in the biased configuration 136 and the neutral configuration 138 in connection with the return electrode 32b and the active electrode 32a, respectively. As demonstrated in various examples, the conductive clip 24 may provide for a conductive connection between each of the control terminals 26 and the control circuit 30. As demonstrated in FIGS. 5A and 5B, control circuit 30 may correspond to a printed circuit board and may include the control terminals 26 on a first side 30a opposite the user inputs 46 of the user interface 44 on a second side 30b. As previously illustrated in FIGS. 1-3, the user inputs 46 may correspond to one or more buttons 160 that may protrude through corresponding interface apertures 162 formed through the second side 14b of the housing 14. In this configuration, the actuation of the buttons 160 may control various operations of the control circuit 30, which may be controlled by a control console 164 via a wired control interface 166 (see FIG. 9). Opposite the buttons 160 of the user interface 44 on the first side 30a of the control circuit 30, the control terminals 26 may conduct driving signals output from the control console 164 via the control circuit 30 to various forms of electrosurgical therapy, ablation, coagulation, resection, etc. with the distal electrode tip 34.

As illustrated in FIG. 6A, the conductive clip 24 may include a mounting aperture 170 that may receive a fastener through the corresponding first side wall 122. In this configuration, the fastener may structurally and conductively couple the conductive clip 24 to the corresponding control terminals 26 of the control circuit 30. As shown, the first side wall 122 may include a straight or flat section through which the mounting aperture may extend and against which the control terminals 26 may engage an enlarged conductive surface area when compared to neighboring curved sections. The flat portion The conductive clip 24 may be securely affixed to the control circuit 30 and may further provide for the conductive connection to each of the tubes 22a, 22b in the biased configuration 136 and the neutral configuration 138.

Referring now to FIGS. 7A and 7B, in various implementations, the disclosure may further provide for the fluid fitting 40, which may be formed as an adapter body 178. As previously discussed, the fluid fitting may provide for a sealed fluid connection and interface between the inner tube 22a and a tubular conduit 180 through which fluids may be aspirated through the central lumen 60 or supplied to the distal electrode tip 34. As best demonstrated in FIG. 7B, the adapter body 178 of the fluid fitting 40 may extend along the longitudinal axis A_{L} and form a barbed end portion 182 on a first proximal side and an opposing receiving end portion 184 configured to receive a threaded proximal end 188 of the inner tube 22a. As demonstrated by the hidden lines in FIGS. 7A and 7B, a coupling lumen 190 may be formed through the adapter body 178 and may taper from the receiving end portion 184 to the barbed end portion 182. In this configuration, the threaded proximal end 188 may readily engage a receiving flange 192 leading to the coupling lumen 190. Once partially inserted, the threaded end portion 188 of the inner tube 22a may rotationally engage the coupling lumen 190 along a tapered portion 194. As the threaded proximal end 188 is further rotated and extends into the coupling lumen 190, the decreased proportions of the tapered portion 194 formed by the adapter body 178 may allow the threaded proximal end 188 to pass through a material forming the adapter body 178 to fluidically seal the threaded proximal end 188 into the receiving end portion 184. Further, as demonstrated in FIG. 7A, the barbed end portion 182 may readily engage the tubular conduit 180 to complete the sealed fluidic transmission passage to or from the surgical apparatus 10.

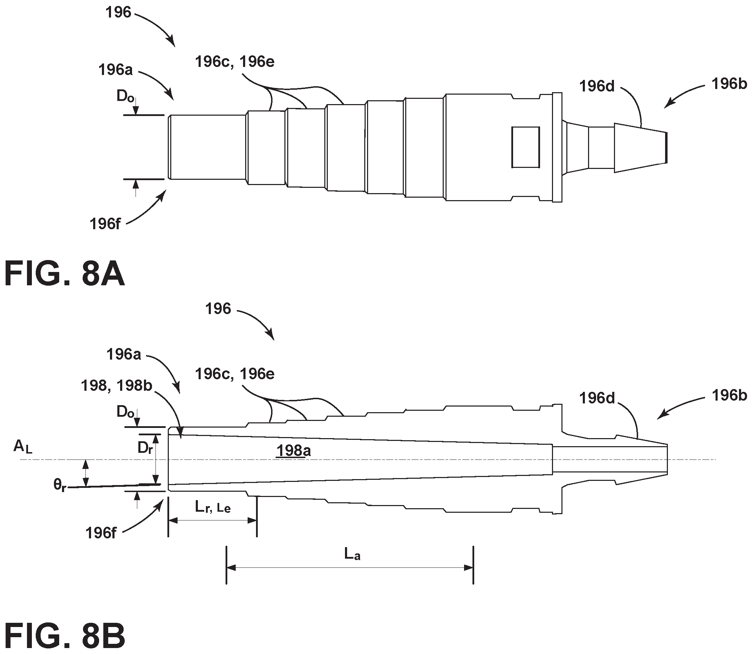

Referring now to FIGS. 8A and 8B, side views of a barbed suction adapter or coupling adapter 196 are shown demonstrating a first coupler 196a and a second coupler 196b positioned on opposing sides. In various implementations, the couplers 196a, 196b may be configured to connect to the tubular conduit 180 and a suction line (not shown). The coupling adapter 196 is shown in connection with the conduit 180 in FIG. 1 and may interconnect the suction line (e.g. associated with a surgical suite) with the conduit 180. Accordingly, the suction or coupling adapter 196 may correspond to a reducing adapter configured to interconnect a first inner diameter of the first of the conduit 180 with a second inner diameter of the suction line. In the example shown, the second inner diameter of the suction line may be greater that the first inner diameter. Accordingly, a plurality of first tapered barbs 196c of the first coupler 196a may be larger in diameter than at least one barb 196d of the second coupler 196b. In this configuration, the barbs 196c, 196d of the couplers 196a, 196b may fluidly interconnect the central lumen 60 extending through the tubular conduit 180 with the suction line without requiring permanent fixation with an adhesive. In this configuration, the surgical apparatus 10 may be readily connected to the suction line to improve the ease of utilization.

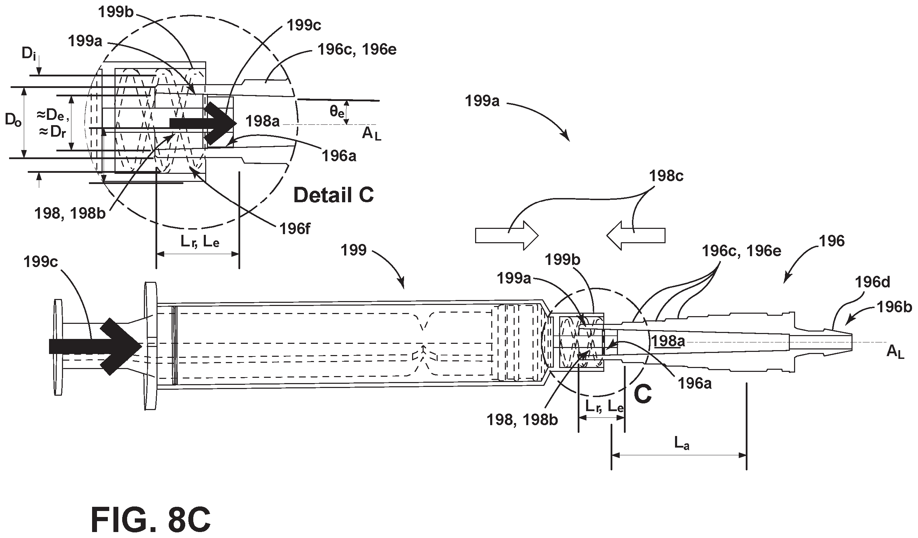

As best shown in FIG, 8B, the first coupler 196a or first hose coupler, the exterior surface and corresponding plurality of barbs 196c may extend in an expanding tapered stair-step 196e forming an increasing tapered outside diameter. Further, a reducing tapered portion 198 of a coupling passage 198a may decrease in diameter opposite the expanding tapered stair-step 196e along a common length Lₐ of the adapter 196. In general, the reducing tapered portion 198 may extend along a receiving angle θᵣ as later discussed in reference to FIG. 8C. In this configuration, the first coupler 196a may provide for a secure connection with suction line having a larger diameter than the tubular conduit 180. Further, the gradual reduction of the reducing tapered portion 198 may decrease the inner diameter of coupling passage 198a and reduce the proportions of the coupling passage 198a to accommodate the reduced diameter of the second coupler 196b. In this way, the barbed suction adapter 196 may ensure the surgical apparatus 10 is consistently and securely connected to the suction line.

FIG. 8C demonstrates additional details of a female coupler 198b that may be formed by the first coupler 196a. As shown, the female coupler 198b may be configured to receive a tapered male connector 199a demonstrated incorporated in a syringe 199. As shown, the tapered male connector 199a may form an engagement angle θₑ. In the particular example shown, the male connector 199a may further include a threaded fitting 199b configured to lock the male connector 199a to various compatible female couplers. The threaded fitting 199b may form an inside fitting diameter Dᵢ within which an outside coupling diameter Dₒ of the first coupler 196a may extend to securely engage the male connector 199a. Further, a receiving diameter Dᵣ of the first coupler 196a may be proportioned in a tapered or drafted configuration to sealably receive an engaging diameter Dₑ of the male connector 199a. In various implementations, the female coupler 198b may be configured to securely engage the male connector 199a and seal along a tapered profile 199b along an engagement length Lᵣ of the tapered portion 198 of the coupling passage 198a. Detail C shown in FIG. 8C demonstrates the engagement between the exemplary tapered male connector 199a in fluid connection with the female coupler 198b.

Referring now to FIGS. 8A-8C, the female coupler 198b may form the receiving angle θᵣ at least along the receiving length Lᵣ, which may correspond to complementary engagement angle θₑ and length Lₑ of the tapered male connector 199a. The remainder of the common length Lₐ may vary in angle and proportions while maintaining the coupling passage 198a between the first coupler 196a and the second coupler 196b. In this configuration, at least part of the tapered portion 198 of the coupling passage 198a may form a fluid seal in connection with the tapered male connector 199a. In various implementations, the fluid seal may be engaged or maintained by applying a manual force in a coupling direction 198c along the longitudinal axis A_{L} of the suction adaptor 196. In this way, the tapered male connector 199a of the syringe 199 or similar fluid supply devices may sealably engage the tapered portion 198 of the coupling passage 198a and inject fluid into the coupling passage 198a as demonstrated by annotated arrow 199c. In various cases, the fluid may be supplied or forced through the coupling passage 198a to deliver a fluid through the suction adaptor 196 and may be particularly beneficial in clearing clogs that may form within the coupling passage 198a or further along a fluidic path formed in part by the suction adaptor.

Still referring to FIGS. 8A-8C, in some implementations, the first coupler 196a may form an engaging tip 196f through which the coupling passage 198a may extend along the tapered portion 198. The engaging tip 196f may define the outside coupling diameter Dₒ and an inside receiving diameter Dᵣ that may provide for the female coupler 198b to fluidically couple to the tapered male connector 199a. As previously discussed, the threaded fitting 199b may form an inside fitting diameter Dᵢ within which an outside coupling diameter Dₒ may extend in the coupled configuration shown in FIG. 8C. Accordingly, the outside coupling diameter Dₒ formed by the engaging tip 196f of the first coupler 196a may be less than the inside fitting diameter Dᵢ, allowing for a clearance fit with the threaded fitting 199b of the male connector 199a. Additionally, receiving diameter Dᵣ of the first coupler 196a may be proportioned in a tapered or drafted configuration approximately along the engagement angle θₑ to receive the engaging diameter Dₑ of the male connector 199a.

In various implementations, the engagement between the male connector 199a and the female coupler 198b may form a tapered fit. Accordingly, the receiving diameter Dᵣ of the first coupler 196a may be slightly less than the engaging diameter Dₑ of the male connector 199a to form a locking friction fit. Additionally, the receiving angle θᵣ may correspond closely to or be approximately equal to the engagement angle θₑ of the tapered male adapter 199a. In this configuration, the female coupler 198b and the tapered male coupling 199a may form mating conical engagement surfaces with a common taper angle. For example, the receiving angle θᵣ and the engaging angle θₑ may be approximately equal and range from 0.5° to 10°, 1° to 5°, or approximately 1.25° to 3°. In some implementations, the receiving angle θᵣ may be approximately 1.5° to 2°, 1.6° to 1.8°, or approximately 1.7°. Additionally, the receiving length Lᵣ may be configured to receive a portion of the engaging length Lₑ sufficient to achieve the fluidic coupling between the female coupler 198b and the tapered male coupling 199a. For example, the length may vary from about 2 mm to 15 mm, 7 mm to 11 mm, or approximately 7.5 mm to 10.5 mm depending on the application. It shall be understood that the dimensions of the female coupler 198b and the tapered male connector 199a may vary depending on the specific application. However, the relationships between the various features depicted and described may be maintained to ensure the effective fluid coupling between the male adapter 199a and the female coupler 198b.

Referring now to FIGS. 9A-9C, the conductive clip 24 is demonstrated with several mounting features that may be implemented alternatively or in combination with the features demonstrated in FIGS. 6A-6C. Accordingly, the features of the conductive clip 24 demonstrated in the examples shown in FIGS. 9A-9C may be referred to with like reference numerals and similar terminology for clarity. As generally shown, the conductive clip 24 may be configured to bend along the curved spring portion 126 between the biased configuration 136 and the neutral configuration 138. In this way, the conductive clip 24 may provide for a secure conductive connection to the differing first diameter D1 and the second diameter D2 of the active electrode 32a and the passive or return electrode 32b, respectively. The retention of the electrodes 32 may also similarly be provided by the first side wall 122 and second side wall 124 forming the U-shaped body 120 and extending to the first arc 132 and second arc 134. As demonstrated in FIGS. 9B and 9C, the first arc 132 and the second arc 134 may protrude between the first side wall 122 and the second side wall 124 and at least partially enclose about a perimeter of each of the electrodes 32. As previously discussed, the dimensions and proportions of the conductive clip 24 may vary depending on the application. For example, the first diameter D1 of the inner tube 22a may vary from approximately 1 mm to 4 mm. The second diameter D2 of the outer tube 22b may vary from approximately 2 mm to 7 mm. Accordingly, the conductive clip 24 may conductively engage tubular bodies with effective diameters having ratios of 3:4, 2:3, 1:2, or 1:3 between the first diameter D1 and the second diameter D2.

In the example demonstrated in FIGS. 9A-9C, the conductive clip 24 may include at least one interior clip leg 200 that may extend inward toward the curved spring portion 126 of the U-shaped body 120 from at least one of the first arc 132 and the second arc 134. As shown, the at least one interior clip leg 200 may comprise a first clip leg 200a extending from the first arc 132 and a second clip leg 200b extending from the second arc 134. Each of the clip legs 200a, 200b may form opposing curved sections 202 that may extend over diverging portions 204 along an intermediate portion 206 to converging portions 208 along opposing distal portions 210. The opposing diverging portions 204 and converging portions 208 may approximately form a convex region 212 at least partially enclosed by opposing conductive connection surfaces 214. More specifically, the conductive surfaces 214 may include a first arc 214a and a second arc 214b that bow outwards from each other along the diverging portions 204 and approach toward and intersection trajectory along the converging portions 208.

In operation, the interior clip legs 200 forming the convex region 212 may provide for opposing conductive connection surfaces 214 that may be spring-biased by the extension of the interior clip legs 200 from the corresponding first arc 132 and second arc 134. In a maiden configuration with the conductive clips 24, the interior clip legs 200 may bend along the corresponding arcs 132, 134 to align the conductive connection surfaces 214 with the perimeter walls of the electrodes 32. In this way, the conductive clip 24 may provide for a variety of features that may assist in the structural and conductive coupling between the conductive connection surfaces 214 and the electrodes 32.

Referring again to FIGS. 4 and 5 while considering the conductive clips 24 of FIGS. 6 and 9, in some implementations, the surface of the return electrode 32 may include a lateral slot 216 that may correspond to an at least partially cutaway portion that may decrease the second diameter D2 aligned with the conductive clip 24. The lateral slots 216 may be formed along the dorsal surface 82a and may be approximately opposite the control circuit. The lateral slot 216 may extend partially or completely through a side wall of the return electrode 32b while maintaining the first insulating layer 66 between the electrodes 32. In some implementations, the lateral slots 216 may correspond to relief cuts that may be approximately 0.25-0.7 mm deep on each side. Accordingly, the overall relief depth may range from approximately 0.5-1.4 mm. The available depth of the relief cut may be based on the material thickness of the outer shaft tube 22b or shaft and at least a portion of the first insulating layer 66 may be maintained to ensure that the electrodes 32 are not shorted. In this configuration, the second diameter D2 of the return electrode 32b may be limited along the conductive interface with the conductive clip 24 to further ensure effective conductive contact between the conductive clip 24 and the return electrode 32b.

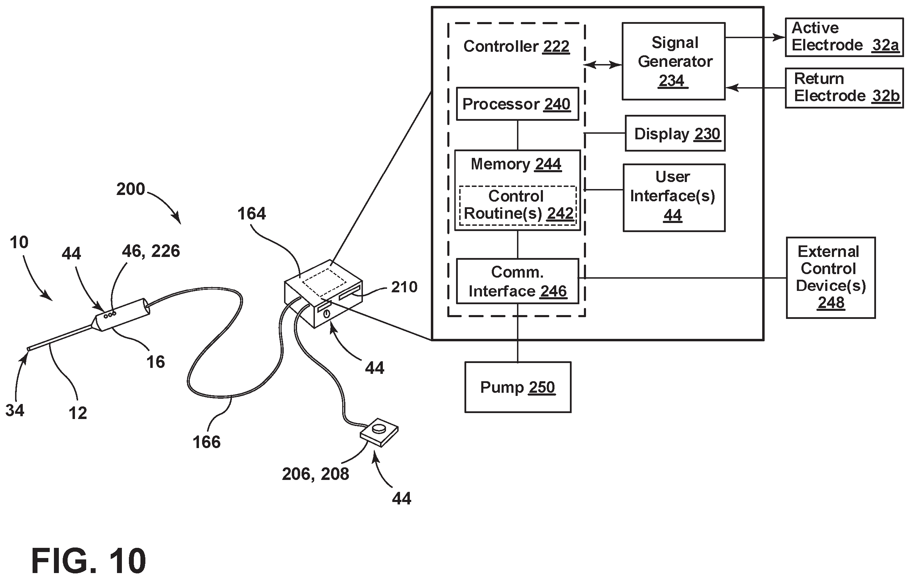

Referring now to FIG. 10, a surgical system 220 comprising the surgical apparatus 10 in an operating configuration is shown. As previously discussed, the surgical apparatus 10 may be in communication with a controller 222 that may control the delivery of signals to the active electrode 32a and/or control a fluid flow rate (e.g., an aspiration rate) through the lumen 20. In operation, the controller 222 may receive inputs via a user interface 44, which may be distributed among the control console 164 as well as one or more external control devices 226. The external control devices 226 may correspond to one or more electronic or electromechanical buttons 160, triggers, or pedals incorporated on the handle portion 16 of the surgical apparatus 10, one or more foot pedals 228, and additional peripherals and devices communicatively connected to the control console 164. The user interface 44 may include one or more switches, buttons, dials, and/or displays, which may include soft-key or touchscreen devices incorporated in a display device 230 (e.g., liquid crystal display [LCD], light emitting diode [LED] display, cathode ray tube [CRT]). In response to inputs received from the user interface 44, the controller 222 may activate or adjust the settings of the control signals communicated to the surgical apparatus 10. The control signals generated by control console 164 may be controlled by a signal generator 234 configured to generate periodic or RF signals that induce a treatment field (e.g., an electric or RF field) in response to control instructions (e.g., timing signals, amplification) communicated from the controller 222. The control signals may be communicated from the signal generator 234 of the control console 164 to the surgical apparatus 10 via the wired control interface 166.

The conductive connectors of the wired control interface 166 may be connected to the active or supply electrode 32a to transmit the output control signal Tx and connected to the return electrode 32b to receive a return signal Rx. The return signal Rx may be monitored by the controller 222 to provide closed-loop feedback to adjust the control signal Tx. The control signal Tx from the signal generator 234 may correspond to an AC driving signal generated in response to time-modulated signals from a processor 240 of the controller 222. The AC driving signal may induce the treatment or ablation field in the form of RF energy. The modes of operation of the surgical apparatus 10 may be controlled by adjusting the amplitude of the voltage and timing of the signal modulation that instructs the signal generator 234 to generate RF signals. Accordingly, by adjusting the voltage potential and the frequency or timing characteristics of the AC driving signal output from the signal generator 234, the controller 222 may control the operation of the surgical apparatus 10 in response to inputs received via the user interface 44. In some embodiments, the controller 222 may be configured to activate one or more preset modes (e.g., ablation, coagulation) and the associated power levels or frequencies as presets in response to inputs received from the user interface 44.

The processor 240 of the controller 222 may be implemented as a microprocessor, microcontroller, application-specific integrated circuit (ASIC), or other circuitry configured to perform instructions, computations, and control various input/output signals to control the surgical system 220. The instructions and/or control routines 242 of the system 90 may be accessed by the processor 240 via a memory 244. The memory 244 may comprise random access memory (RAM), read only memory (ROM), flash memory, hard disk storage, solid state drive memory, etc. The controller 222 may incorporate additional communication circuits or input/output circuitry. In an exemplary embodiment, a communication interface 246 of the controller 222, may include digital-to-analog converters, analog-to-digital converters, digital inputs and outputs, as well as one or more peripheral communication interfaces or busses. The peripheral communication interfaces of the communication interface 246 may be implemented with by various communication protocols, such as serial communication (e.g., CAN bus, I2C), parallel communication, network communication (e.g., RS232, RS485, Ethernet), wireless network communication (Wi-Fi, 802.11). In some examples, the controller 202 may be in communication with one or more external devices 248 (e.g., control devices, peripherals, servers) via the communication interface 246. Accordingly, the control console 164 may provide for communication with various devices to update, maintain, and control the operation of the surgical system 220.

Though not pictorially illustrated in the figures, a pump 250 or aspiration pump may be connected via one or more fluid conduits in connection with the lumen 20 to effectuate fluid transfer via a fluid transmission path FP comprising the aspiration aperture(s) or port(s) 22, the transition passage, and the lumen 20. The pump 250 may be controlled via the user interface 44 of the controller 222 to adjust a flow rate or intensity of the fluid transfer. The pump 250 may be implemented with a variety of pumping technologies (e.g., peristaltic, reciprocating) and may vary in fluid transfer capacity based on the application of the surgical apparatus 10.

According to some aspects of the disclosure, an electrosurgical probe includes an elongated probe extending along a longitudinal axis and including an inner tube extending through an outer tube. The inner tube forms a lumen extending along a longitudinal axis from a proximal tube portion to a distal tube portion. The distal tube portion forms an active electrode, and the proximal tube portion of the outer tube includes a plurality of mating apertures. A handle portion includes a probe coupling configured to receive the proximal tube portion, while the probe coupling includes a plurality of protrusions that engage the mating apertures of the outer tube portion.

According to various aspects, the disclosure may implement one or more of the following features or configurations in various combinations:
- the engagement of the protrusions with the mating apertures constrains the elongated probe in connection with the handle portion;
- the engagement of the protrusions with the mating apertures orients the elongated probe to the handle in a predetermined circumferential orientation;
- the protrusions comprise at least one asymmetric feature that limits the engagement of the protrusions with the mating apertures to the predetermined circumferential orientation;
- the handle portion forms a housing comprising a first side and a second side separated along a parting line;
- the first side and the second side define opposing sides of the probe forming a clamshell assembly;
- the protrusions are formed on the first side and the second side;
- the housing forms a shell comprising a plurality of ribs extending inward toward the longitudinal axis and defining one or more of the protrusions;
- at least one of the ribs is aligned transverse to the longitudinal axis;
- at least one of the ribs forms a trough aligned with the longitudinal axis;
- the trough comprises opposing side walls that engage the mating apertures on opposing sides of the outer tube;
- the plurality of mating apertures of the outer tube comprise a plurality of slots;
- at least one of the plurality of slots is oriented transvers to the longitudinal axis;
- the mating apertures extend along opposing surfaces of the outer tube and form at least one asymmetric feature; and/or
- at least one asymmetric feature includes at least one of the mating apertures being different in geometry, size, or number on one of the opposing sides.

According to another aspect of the disclosure, a conductive clip for an electro-surgical apparatus includes a U-shaped body with a thickness and a width defining a first side wall that deflects angularly relative to a second side wall along a curved spring portion. The first side wall extends to a first end portion forming a first arc extending toward the second side wall, and the second side wall extends to a second end portion forming a second arc extending toward the first side wall.

According to various aspects, the disclosure may implement one or more of the following features or configurations in various combinations:
- at least one of the first arc and the second arc extend to an interior compression leg that extends into a trough forming the U shaped body;
- the compression leg is interposed between an opposing one of the first side wall and the second side wall and compresses toward the one of the first side wall and the second side wall from which the compression leg extends;
- a first leg extending from the first arc and a second leg extending from the second arc, wherein the first leg and the second leg extend into a trough formed by the U-shaped body and form opposing conductive contact surfaces;
- the first leg and the second leg form opposing convex profiles defining the conductive contact surfaces therebetween;
- the convex profiles form an elliptical opening and the first arc and the second arc form converging portions of the elliptical opening;
- the first arc defines a first radius R1 and the second arc defines a second radius R2, wherein the second radius is larger than the first radius;
- the ratio of R1 to R2 is greater than or equal to one of: 4:5, 3:4, 2:3, 1:2, 1:3, or 1:4;
- the first arc extends circumferentially to a first arc length enclosing at least two-thirds of an interior passage formed by the first arc;
- the second arc extends circumferentially to a second arc length enclosing less than two-thirds of an interior passage formed by the second arc;
- the conductive clip is configured to flex along the spring portion from a neutral configuration to an expanded configuration;
- the first arc is positioned adjacent the second arc in the neutral configuration;
- the first arc is biased toward and abuts the second arc in the neutral configuration;
- a first spacing between the first side wall and the second side wall in the neutral configuration extends along a distance defined by a combination of a first diameter of the first arc and a second diameter of the second arc;
- the second arc further comprises a curved or crimped distal end configured to tangentially engage the cylindrical structure comprising the second diameter;
- the first side wall comprises a generally flat section comprising a mounting aperture; and/or
- the mounting aperture and the flat section are configured to surface mount to a control circuit of the electro-surgical apparatus.

According to yet another aspect of the disclosure, a fluid fitting for an electrosurgical apparatus includes an adapter body extending along a longitudinal axis from a barbed end portion to a receiving end portion and forming a lumen extending there through along the longitudinal axis. The barbed end portion is configured to engage an interior passage formed by a flexible tube. The receiving end portion forms a cylindrical opening extending along a receiving portion of the lumen, wherein the cylindrical opening is configured to receive a threaded proximal end portion a conductive rod forming a portion of a conductive circuit for the electrosurgical apparatus.

According to various aspects, the disclosure may implement one or more of the following features or configurations in various combinations:
- the conductive rod forms an aspiration lumen extending from within a housing of the electrosurgical apparatus and to a distal end portion;
- the conductive rod conducts an electrical signal to a distal probe portion and the aspiration lumen is in fluid connection with one or more aspiration apertures formed in the distal end portion;
- the conductive rod defines an inner conductive tube extending from the housing to the distal probe portion and is in connection an active electrode of the electrosurgical apparatus; and/or
- the inner conductive tube is disposed within an outer conductive tube, and the inner conductive tube is connected to the active electrode, and the active electrode is connected to the outer conductive tube via an insulating spacer.

According to a still further aspect of the disclosure, a coupling adapter fitting for a surgical tool includes a first coupler forming a first side including a plurality of barbs or steps. A second coupler forms a second side opposite the first side along a longitudinal axis. A fluid conduit extends from the first side to the second side, wherein the plurality of barbs or steps increase in an outside diameter along at least a portion of a length of the first coupler.

According to various aspects, the disclosure may implement one or more of the following features or configurations in various combinations:
- the plurality of barbs or steps form an expanding stair-stepped configuration along the length;
- the fluid conduit is formed through a body of the adapter fitting centrally through the first coupler and the second coupler;
- the tapered portion forms a tapered female connector configured to receive a tapered male connector over an engagement length;
- the tapered female connector extends along the engagement length at a taper angle ranging from approximately 0.5° to 10° or 1.25° to 3°;
- the tapered female connector forms an outside diameter, the outside diameter being less than an inside diameter of a coupling portion of the tapered male coupler configured to engage the tapered female adapter to form a fluid coupling;
- the tapered male coupler is connected to a fluid supply device, and the fluid supply device is configured to deliver pressurized fluid through a fluid coupling formed between the tapered female connector and the tapered male connector;
- an inside diameter of a tapered portion of the fluid conduit extending through the first coupler decreases along the portion of the length of the first coupler;
- the decrease in the inside diameter of the tapered portion is sloped toward the longitudinal axis and the increase in the outside diameter is sloped away from the longitudinal axis;
- a wall thickness of the body forming the first coupler expands in thickness as the taper portion slopes away from an expanding stair-stepped portion of the first coupler;
- the second coupler comprises at least one barb;
- the first coupler is configured to engage a suction line having a first line diameter, and the second coupler is configured to engage a tubular conduit of the surgical tool having a second line diameter, wherein first line diameter is greater than the first line diameter; and/or
- at least a portion of the fluid conduit tapers from the first coupler to the second coupler accommodating the second line diameter smaller than the first line diameter.

As used herein, words of approximation such as, without limitation, "approximately," "substantially," or "about" refer to a condition that when so modified is understood to not necessarily be absolute or perfect but would be considered close enough to those of ordinary skill in the art to warrant designating the condition as being present. Further, the lack of such modifying terms does not otherwise require strict interpretation of the corresponding value or property. Instead, the extent to which the associated interpretation varies will depend on how great a change can be instituted and still have one of ordinary skill in the art recognize the modified feature as having the required characteristics or capabilities of the unmodified feature. Such determinations may vary considerably depending on the technological field based on the corresponding equivalency associated with the described operation or property. In general, but subject to the preceding discussion, a numerical value herein that is modified by a word of approximation such as "approximately" may vary from the stated value by ±0.5%, ±1%, ±2%, ±3%, ±4%, ±5%, ±10%, ±12%, or ±15%.

Any element in a claim that does not explicitly state "means" for performing a specified function or "step" for performing a specified function, should not be interpreted as a "means" or "step" clause as specified in 35 U.S.C. § 112.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents.

## Claims

1. An electrosurgical apparatus (10) comprising:
an elongated probe (12) extending along a longitudinal axis (A_{L}) and comprising an inner tube (22a) extending through an outer tube (22b), wherein the inner tube (22a) forms a lumen (60) extending along a longitudinal axis (A_{L}) from a proximal tube portion to a distal tube portion, the distal tube portion forming an active electrode (32a) and wherein the proximal tube portion of the outer tube (22b) comprises a plurality of mating apertures (80); and
a handle portion (16) comprising a probe coupling configured to receive the proximal tube portion, wherein the probe coupling comprises a plurality of protrusions (84) that engage the mating apertures (80) of the outer tube portion (22b).

2. The electrosurgical apparatus (10) according to claim 1, wherein the engagement of the protrusions (84) with the mating apertures (80) constrains the elongated probe (12) in connection with the handle portion (16).

3. The electrosurgical apparatus (10) according to claim 1 or 2, wherein the engagement of the protrusions (84) with the mating apertures (80) orients the elongated probe (12) to the handle (16) in a predetermined circumferential orientation.

4. The electrosurgical apparatus (10) according to claim 3, wherein the protrusions (84) comprise at least one asymmetric feature that limits the engagement of the protrusions (84) with the mating apertures (80) to the predetermined circumferential orientation.

5. The electrosurgical apparatus (10) according to any one of claims 1-4, wherein the handle portion (16) forms a housing (14) comprising a first side (14a) and a second side (14b) separated along a parting line (42).

6. The electrosurgical apparatus (10) according to claim 5, wherein the first side (14a) and the second side (14b) define opposing sides of the probe (12) forming a clamshell assembly.

7. The electrosurgical apparatus (10) according to claim 5 or 6, wherein the protrusions (84) are formed on the first side (14a) and the second side (14b).

8. The electrosurgical apparatus (10) according to any one of claims 5-7, wherein the housing (14) forms a shell comprising a plurality of ribs (86) extending inward toward the longitudinal axis (A_{L}) and defining one or more of the protrusions (84).

9. The electrosurgical apparatus (10) according to claim 8, wherein at least one of the ribs (86) is aligned transverse to the longitudinal axis(A_{L}).

10. The electrosurgical apparatus (10) according to claim 8 or 9, wherein at least one of the ribs (86) forms a trough (100) aligned with the longitudinal axis (A_{L}).

11. The electrosurgical apparatus (10) according to claim 10, wherein the trough (100) comprises opposing side walls (98) that engage the mating apertures (80) on opposing sides of the outer tube (22b).

12. The electrosurgical apparatus (10) according to any one of claims 1-11, wherein the plurality of mating apertures (80) of the outer tube (22a) comprise a plurality of slots (92, 94).

13. The electrosurgical apparatus (10) according to claim 12, wherein at least one of the plurality of slots (92, 94) is oriented transvers to the longitudinal axis (A_{L}).

14. The electrosurgical apparatus (10) according to any one of claims 1-13, wherein the mating apertures (80) extend along opposing surfaces of the outer tube (22b) and form at least one asymmetric feature and the at least one asymmetric feature includes at least one of the mating apertures (80) being different in geometry, size, or number on one of the opposing sides.

15. A method of assembling an electrosurgical apparatus (10) of claim 1, the method comprising:
receiving the elongated probe (12) in the handle portion (16);
positioning the proximal tube portion within the probe (12) coupling of the handle portion (16), the handle portion (16) comprising a housing (14) having a first side (14a) and second side (14b); and
bringing the first side (14a) and the second side (14b) together into an assembled configuration, wherein a plurality of protrusions (84) of the housing (14) engage the mating apertures (80) of the outer tube (22b) to secure the elongated probe (12) to the handle portion (16) to constrain the elongated probe (12) axially and rotationally in a predetermined position and orientation relative to the handle portion (16).
